(19) Europäisches Patentamt
European Patent Office
Office européen des brevets

(11) Publication number: **0 192 421**
**A2**

(12) # EUROPEAN PATENT APPLICATION

(21) Application number: 86300991.6

(22) Date of filing: 13.02.86

(51) Int. Cl.[4]: C 07 C 153/00
C 07 C 149/437, C 07 C 125/067
C 07 D 307/86

(30) Priority: 14.02.85 US 701682

(43) Date of publication of application:
27.08.86 Bulletin 86/35

(84) Designated Contracting States:
AT BE CH DE FR GB IT LI LU NL SE

(71) Applicant: E.I. DU PONT DE NEMOURS AND COMPANY
1007 Market Street
Wilmington Delaware 19898(US)

(72) Inventor: Fuchs, Julius Jakob
1104 Greenway Road Forwood
Wilmington Delaware 19803(US)

(74) Representative: Hildyard, Edward Martin et al,
Frank B. Dehn & Co. Imperial House 15-19 Kingsway
London WC2B 6UZ(GB)

(54) **Process for the preparation of N-methyl carbamates.**

(57) N-methylcarbamate compounds such as oxamyl, aldicarb, carbaryl, and carbofuran can be prepared by reacting the corresponding oxime or phenol in aqueous suspension with phosgene and methylamine, simultaneously, in the presence of an alkaline base.

EP 0 192 421 A2

Croydon Printing Company Ltd.

# "Process for the preparation of N-methyl carbamates"

This invention concerns a process for the preparation of N-methyl carbamates having insecticidal activity, viz. oxamyl, aldicarb, carbaryl and carbofuran.

U.S. Patent 3,217,037 discloses the production of carbamate compounds by the sequential reaction of the corresponding oxime with phosgene, to form a chloroformate, and then with an amine to provide the desired carbamoyloxime. A process for the production of aldicarb is embraced in this patent as follows:

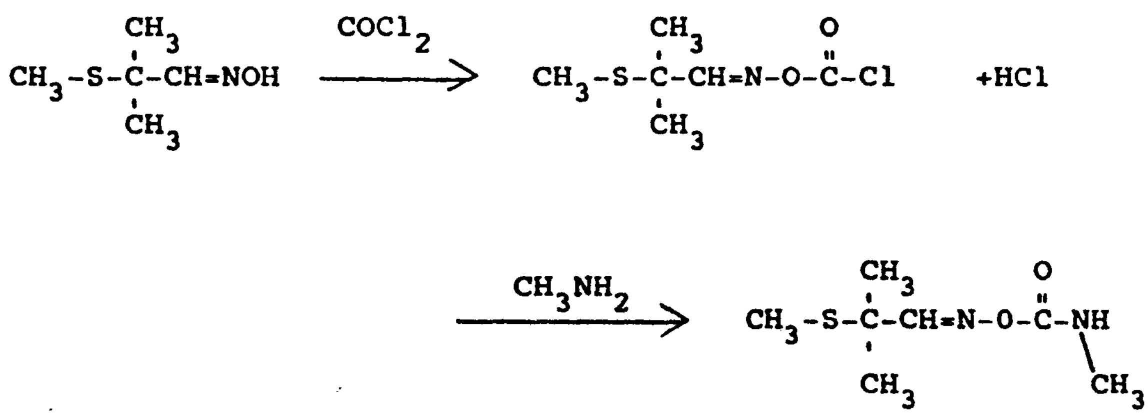


This procedure has the disadvantage of requiring two-steps, which adds considerably to the cost of operation.

U.S. Patent 4,333,882 discloses a process for preparing methomyl by reacting methylthioacetaldoxine, in an aqueous suspension, with gaseous phosgene and aqueous methylamine in a single step. Although this process eliminates the need for a multi-step reaction, the disclosure is limited to production of a single N-methylcarbamate, methomyl.

## Summary of the Invention

The present invention provides a process for preparing an N-methylcarbamate compound of the Formula (I)

$$R-O-\overset{O}{\overset{\|}{C}}NHCH_3 \qquad (I)$$

where R is $(CH_3)_2N-\overset{O}{\overset{\|}{C}}-\underset{SCH_3}{\underset{|}{C}}=N-$ , $CH_3S-\overset{CH_3}{\overset{|}{\underset{CH_3}{\underset{|}{C}}}}-CH=N-$ ,

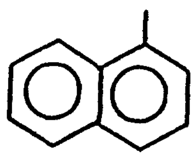 , or 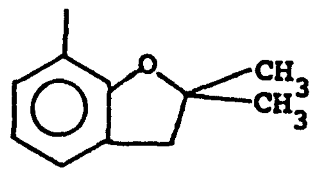

comprising treating an aqueous suspension of a compound of the formula R-OH, where R is as defined above, simultaneously with gaseous phosgene and methylamine in the presence of an alkaline base providing a pH of 8-12 at a temperature of 0-40°C.

DESCRIPTION OF THE INVENTION

The process of the present invention is represented by Equation 1 in which a compound of the formula R-OH (II), where R is

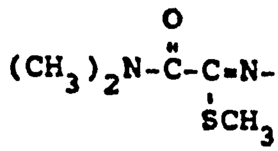 ,

(a)

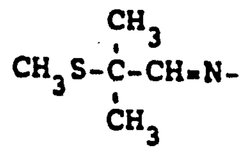 ,

(b)

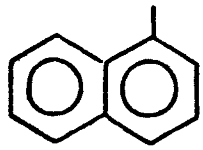 , or

(c)

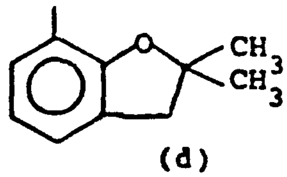

(d)

is reacted with gaseous phosgene and methylamine, in a single step, in a basic aqueous medium, to provide an N-methylcarbamate of Formula I.

Equation 1

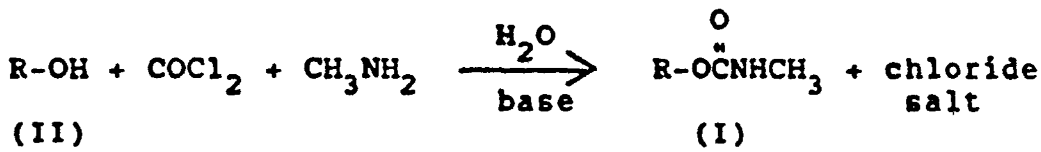


(II) (I)

The compounds of Formula (I) are useful insecticides whose common chemical names, according to R values (a)-(d), are oxamyl, aldicarb, carbaryl, and carbofuran, respectively. The starting compounds of Formula (II) are known in the art and have been taught as intermediates for the production, by other processes, of the above mentioned N-methylcarbamate insecticides. The preferred practice of our invention will now be described.

In the conduct of the present invention, the compound of Formula (II) is suspended in water. The suspension is then treated with an aqueous solution of inorganic alkaline base and methylamine, while phosgene is simultaneously bubbled through the suspension. The alkaline base is preferably sodium or potassium hydroxide and is present in sufficient quantity to

maintain the suspension at a pH of 8-12, preferably about 9-10, during the course of the reaction. This pH value can normally be attained by using 1-4 equivalents of alkaline base, preferably 2-3 equivalents, based on the amount of Formula (II) compound initially present. The phosgene is added in excess, and the amount consumed is determined by the amount of methylamine and alkaline base added.

The temperature of the reaction, which is exothermic, is maintained between 0-40°C, and preferably between 20-25°C. The pH is maintained within the prescribed limits by adjusting the flow of phosgene or the addition rate of the solution of base and methylamine. From 1-3 moles of methylamine, preferably about 2 moles of methylamine, are added to the suspension for each mole of Formula (II) compound initially present. The reaction time during which the phosgene and amine/base solution are added to the suspension, depends on such factors as the reaction temperature and reactant concentrations, but generally will range from thirty minutes to 6 hours. A time of one hour is preferred and can best be achieved when the temperature is about 20-25°C.

In the conduct of the reaction, it is preferred to first adjust the pH of the suspension of the Formula (II) compound to the desired level by starting the addition of the base/methylamine solution. When the proper pH level is attained, the flow of phosgene is begun, and the solution and phosgene are thereafter added simultaneously. When all of the base/methylamine solution has been added, the flow of phosgene can be stopped, or can be continued, with agitation, until the pH drops to between 7-8. The reaction product of Formula (I) can be recovered by filtration, or by extraction in an organic solvent such as methylene chloride followed by evaporation of the solvent.

## Example

S-methyl 1-(dimethylcarbamoyl)-N-hydroxythioformimidate (81.1 g) was suspended in 500 ml water and the pH of the suspension adjusted to 9.5 with the dropwise addition of an aqueous base/amine solution made up of 120 g 50% aqueous NaOH solution, 240 ml water and 77.5 g of a 40% aqueous solution of methylamine. Phosgene was then sparged in with good agitation, maintaining the pH at 9.5 by simultaneous addition of the aqueous base/amine solution, while the temperature was maintained at 20-25°C. When all of the aqueous base/amine solution was added, over a period of approximately one hour, 79 g of phosgene had been charged, and the phosgene feed was stopped. Agitation was continued for 30 minutes, after which time the pH had dropped to 7.9. The reaction mass was then extracted in three portions each of 500 ml methylene chloride. The combined extracts were dried with magnesium sulfate and evaporated under vacuum to give 112.5 g of a clear, viscous oil which, when triturated with 100 ml benzene, crystallized to 103.9 g of white solids, m.p. 104.5- 106.5, containing 98.2% S-methyl 1-(dimethylcarbamoyl)-N-[(methylcarbamoyl)oxy]thioformimidate("Oxamyl"), 0.3% starting oxime and 0.2% dimethylurea.

Claims:

1. A process for preparing an N-methylcarbamate compound of the Formula (I)

$$R-O-\overset{O}{\overset{\|}{C}}NHCH_3 \qquad (I)$$

where R is $(CH_3)_2N-\overset{O}{\overset{\|}{C}}-\underset{SCH_3}{C}=N-$ , $CH_3S-\underset{CH_3}{\overset{CH_3}{C}}-CH=N-$ ,

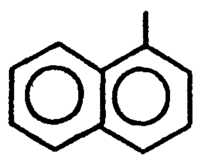 , or 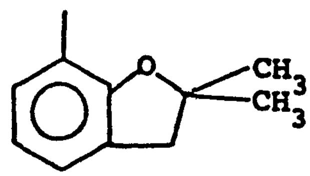

comprising treating an aqueous suspension of a compound of the formula R-OH, where R is as defined above, simultaneously with gaseous phosgene and methylamine in the presence of an alkaline base providing a pH of 8-12 at a temperature of 0-40°C.

2. A process of Claim 1 in which the base is sodium hydroxide.

3. The process of claim 1 or 2 wherein 1 to 4 equivalents of said alkaline base are employed, based on the amount of Formula (II) compound initially present.

4. The process of claim 3 wherein 2 to 3 equivalents of said alkaline base are employed, based on the amount of Formula (II) compound initially present.

5. The process of any of claims 1 to 4 wherein about 2 moles of methylamine are used for each mole of Formula (II) compound initially present.

6. A process of Claim 2 in which the reaction temperature is 20-25°C.

7. A process of any of claims 1 to 6 in which R is

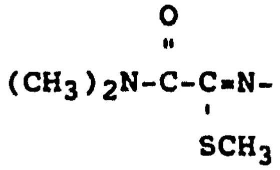

.

8. A process of any of claims 1 to 6 in which R is

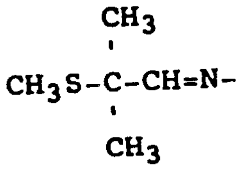

.

9. A process of any of claims 1 to 6 in which R is

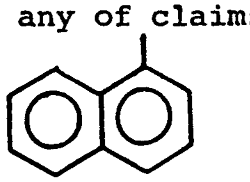

10. A process of any of claims 1 to 6 in which R is